# EUROPEAN PATENT APPLICATION

(11) **EP 3 643 329 A1**
(43) Date of publication of application: **29.04.2020**
(21) Application number: 19213777.6
(22) Date of filing: 12.10.2015
(51) Int. Cl.: A61L 15/32, A61L 27/58, A61F 2/08, A61L 27/24, A61L 27/50

(54) **SUTURELESS REPAIR OF SOFT TISSUE**

(30) Priority: 10.10.2014 AU 2014904054
(62) Divisional of application: 15849293.4
(71) Applicant: Orthocell Limited, Murdoch WA 6150 (AU)
(72) Inventor: ZHENG, Ming Hao, City Beach, W.A. 6015 (AU)
(74) Representative: De Clercq & Partners

(57) **Abstract**

The present invention relates to a sutureless method of repairing soft tissue defects in soft tissue including ligaments such as anterior cruciate ligaments (ACLs). In particular, the present invention relates a sutureless method of repairing soft tissue defect comprising: (i) providing a collagen-containing patch adapted to enclose at least a portion of said soft tissue defect; (ii) contacting said soft tissue defect and/or collagen-containing patch with a sensitizer; (iii) enclosing said soft tissue defect in said collagen-containing patch to produce a bioactive chamber; and (iv) adhering said collagen-containing patch to said soft tissue defect without sutures.

## Description

### Field

The present invention relates to a sutureless method of repairing soft tissue defects in soft tissue including ligaments such as anterior cruciate ligaments (ACLs). The repair includes repairing tears, partial or complete. In particular, the present invention relates to a sutureless method of repairing soft tissue defects comprising adhering a collagen-containing patch to the site of the soft tissue defect using a sensitizer wherein a bioactive chamber is formed around the soft tissue defect which provides a microclimate conducive to the repair of the tissue.

### Background

Tendon tissue, ligament tissue, vascular tissue, dermal tissue and the like are often collectively referred to as soft tissue. Ligaments are specialized connective soft tissues that connect different organs or tissues and attach bone to bone. In the latter case, ligaments provide stability to joints by being flexible enough to allow natural movement of the bones yet also are strong and inextensible to prevent resistance to applied forces. Tendons connect muscle to bone and are capable of withstanding tension. In addition, tendons passively modulate forces during locomotion, providing additional stability with no active work. Their elastic properties allow tendons to store and recover energy at high efficiency. In tendons and ligaments, bundles of collagen fibers are embedded in a connecting matrix made of proteoglycans components. These bundles of collagen fibers provide the load carrying elements. In tendons, the collagen fibers are arranged in nearly parallel formation, thus enabling them to withstand high unidirectional loads. In ligaments, the collagen fibers are arranged in a less parallel formation, thereby enabling them to withstand predominant tensile stresses in one direction and smaller stresses in other directions.

Every year, hundreds of thousands of people sprain, tear, or rupture ligaments in particular in the knee, shoulder, and ankle or suffer from injuries to tendons of the upper and lower extremities, in particular in the shoulder, knee, foot, and ankle. One such ligament often affected by these types of injuries is the anterior cruciate ligament (ACL) of the knee. The ACL serves as a primary stabilizer of anterior tibia translation and as a secondary stabilizer of valgus-varus knee angulation, and is often susceptible to rupture or tear resulting from a flexion-rotation-valgus force associated with sports injuries and traffic accidents. Ruptures or tears often result in: severe limitations in mobility; pain and discomfort; and an inability to participate in sports and exercise. More than 200,000 people in the U.S. alone tear or rapture their ACL each year, leading to costs of approximately $3 billion for ACL reconstructive surgery and extensive rehabilitation. It is widely known that the ACL has poor healing capabilities. Total surgical replacement and reconstruction are required when the ACL suffers a significant tear or rapture resulting in joint instability. The most common practice is to reconstruct a torn ACL by substituting the torn ligament with the patient's own tissue, also known as an autograft. Other options for substitute ligaments include donor tissues from another organism, also known as allografts, as well as synthetic grafts.

Reconstruction of soft tissue disruptions using collagen fibers, biodegradable polymers and composites have been used as well as three-dimensional scaffolds made of protein, hydrogel or collagen. However, generally, all of these procedures require some form of suturing.

A surgical technique that eliminates the need for sutures and avoids collateral tissue damage is an unmet goal for microsurgical repair of ligaments, tendons, vessels and nerves. The placement of sutures in delicate structures like ligaments and tendons inevitably causes ongoing problems. For example, the presence of permanent sutures leads to immunological reactions including inflammation and scarring at the repair site. Suture material also creates irregularities of the endothelium with a consequent prothrombotic effect. In addition, the presence of sutures does not provide an instantaneous water-tight seal as shown by relatively low leak point pressures in comparison to other sealant technologies.

In an attempt to overcome some of the limitations of suture repair of ligaments, tendons, vessels, nerves and the like previous researchers have used rings, clips, glues and laser welding. Some of these methodologies are discussed in Zeebregts et al. (2003), Br J Surg; 90:261-271; however, all of these alternative methods have significant drawbacks related to the substantial foreign body reaction or associated tissue injury that have prevented widespread clinical adoption.

Laser assisted repair has been the subject of research for over 25 years and involves delivery of laser energy to heat the repair site to form a "weld" (Wolf-de Jonge et al. (2004), Eur J Vasc Endovasc Surg., 27:466-476). Since the late 1970's there have been numerous reports of welding of tissue using argon, CO₂, near infrared diode and Nd:YAG lasers (see, for example, Gelli et al. (1997), J. Reconstr. Microsurg., 13:199-205; Jain & Gorisch (1979), Surgery, 85:684-688; Lewis & Uribe (1993), Laryngoscope, 103:850-853; Neblett et al. (1986), Neurosurgery, 19:914-934; Samonte & Fried (1991), Lasers Surg Med., 11:511-516; Serure et al. (1983), Surg. Forum, 34:634-636; Tang et al. (1994), Lasers Surg Med., 14:229-237; Vale et al. (1986), Plast. Reconstr. Surg., 77:759-766). In these reports and many others the laser energy is converted to heat to raise the local tissue temperature and denature extracellular matrix proteins which results in tissue welding and repair.

In spite of the many theoretical benefits of laser welding, the technique has not been adopted clinically. The reasons are many, but heating of the tissues necessarily causes injury and thus, despite all the pre-clinical research in the area of laser welding, application in the clinical realm has not been realized.

Thus, there remains a need for a method of repairing soft tissue defects including defects in tendon tissue, ligament tissue, vascular tissue, dermal tissue and the like which alleviates or at least works towards overcoming the issues identified with using scaffolds and sutures.

### Summary

In a first aspect, the present invention provides a sutureless method of repairing soft tissue defect comprising:
(i) providing a collagen-containing patch adapted to enclose at least a portion of said soft tissue defect;
(ii) contacting said soft tissue defect and/or collagen-containing patch with a sensitizer;
(iii) enclosing said soft tissue defect in said collagen-containing patch to produce a bioactive chamber; and
(iv) adhering said collagen-containing patch to said soft tissue defect without sutures.

In some embodiments the soft tissue defect is in a soft tissue selected from the group consisting of ligament tissue, tendon tissue, venous tissue, arterial tissue and nerve tunnel tissue. In some embodiments the soft tissue defect is in a ligament such as anterior cruciate ligament (ACL).

It will appreciated by those skilled in the art, having read the specification as a whole, that the sensitizer has the effect of absorbing energy from a radioactive or light ray, and transmitting the energy to the collagen-containing patch and/or soft tissue such that the collagen-containing patch adheres or "welds" to the soft tissue being treated.

Non-exhaustive examples of sensitizers that can be used in the present invention include acetophenones, benzophenones, naphthalenes, biacetyl, eosine, rose bengal, pyrenes, anthracenes, phenothiazines, and the like. In some embodiments the sensitizer is rose bengal.

The collagen-containing patch preferably has the following properties:
a) pores that interconnect in such a way as to favour tissue integration and vascularisation;
b) biodegradability and/or bioresorbability so that normal tissue ultimately replaces the scaffold;
c) surface chemistry that promotes cell attachment, proliferation and differentiation;
d) strength and flexibility; and
e) low antigenicity.

The collagen-containing patch will comprise greater than 80% type I collagen. In other embodiments, the collagen-containing patch will comprise at least 85% type I collagen. In still other embodiments the collagen-containing patch will comprise greater than 90% type I collagen.

The collagen fibres or bundles within the collagen-containing patch will comprise a knitted structure. The term "knitted structure" as used herein refers to a structure comprising first and second groups of fibres or bundles where fibres or bundles in the first group extend predominately in a first direction and fibres or bundles in the second group extend predominately in a second direction, where the first and second directions are different to each other and the fibres or bundles in the first group interleave or otherwise weave with the fibres or bundles in the second group. The difference in direction may be about 90°.

In some embodiments, the collagen-containing patch has maximum tensile load strength of greater than 20N. In some embodiments, the collagen-containing patch of the present invention has maximum tensile load strength greater than 25N, 40N, 60N, 80N, 100N, 120N or 140N.

In some embodiments the collagen-containing patch has a modulus of greater than 100 MPa. In other embodiments the collagen-containing patch has a modulus of greater than 200 MPa, 300 MPa, 400 MPa, or 500 MPa.

In some embodiments, the collagen-containing patch has extension at maximum load of less than 85% of the original length.

Most importantly, the collagen-containing patch of the present invention must be sufficiently thick to provide support for the soft tissue under repair; however, not too thick that the ability to adhere the collagen-containing patch to the soft tissue is impaired. Thus, in some embodiments the collagen-containing patch is between 25 µm and 200 µm thick. In some embodiments, the collagen-containing patch is between 30 µm and 180 µm thick. In other embodiments, the collagen-containing patch is between 35 µm and 170 µm thick. In still other embodiments, the collagen-containing patch is between 40 µm and 160 µm thick. In still other embodiments, the collagen-containing patch is between 45 µm and 150 µm thick. In still other embodiments, the collagen-containing patch is between 50 µm and 140 µm thick. In still other embodiments, the collagen-containing patch is between 50 µm and 100 µm thick. Finally, in some embodiments the collagen-containing patch is about 50 µm thick.

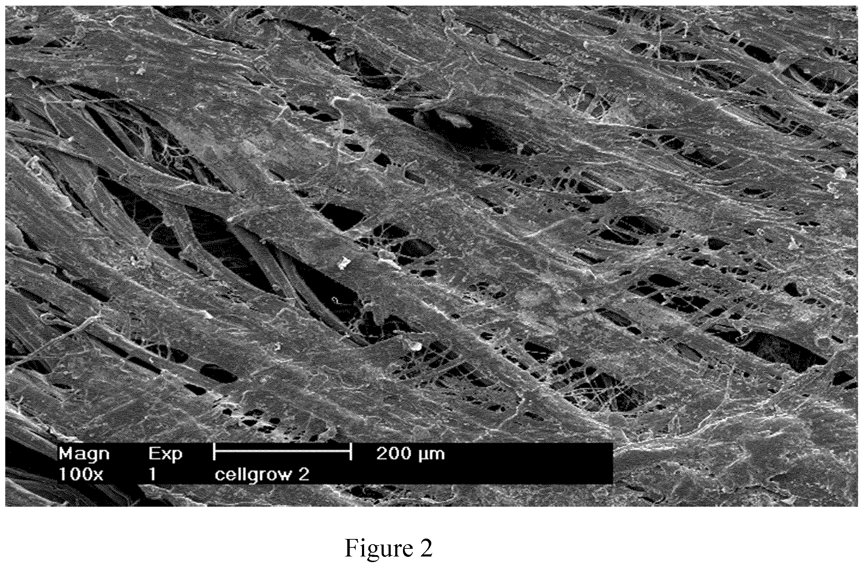

In some embodiments, the collagen-containing patch is adapted by shaping the patch to provide better means of manipulation *in situ.* In some embodiments the collagen-containing patch corresponds to the collagen-containing patch shown in Figure 2.

It will be appreciated by those skilled in the art that the collagen-containing patch can be manufactured using a number of techniques. In some embodiments, the collagen-containing patch is manufactured as follows:
(i) isolating a collagen-containing tissue and incubating same in an ethanol solution;
(ii) incubating the collagen-containing tissue from step (i) in a first solution comprising an inorganic salt and an anionic surfactant in order to denature non-collagenous proteins contained therein;
(iii) incubating the collagen-containing tissue produced in step (ii) in a second solution comprising an inorganic acid until the collagen in said material is denatured; and
(iv) incubating the collagen-containing tissue produced in step (iii) in a third solution comprising an inorganic acid with simultaneous mechanical stimulation for sufficient time to enable the collagen bundles in said collagen-containing tissue to align;
wherein the mechanical stimulation comprises applying tension cyclically to the collagen-containing tissue.

In a second aspect the present invention provides a collagen-containing patch for use in the sutureless repair of soft tissue, wherein said patch is between 25 µm and 200 µm thick, comprises greater than 90% type I collagen, has maximum tensile load strength of greater than 100N, has a modulus of greater than 200 MPa and is capable of enclosing said soft tissue to produce a bioactive chamber.

In some embodiments, the collagen-containing patch for use in the sutureless repair of soft tissue is produced by:
(i) isolating a collagen-containing tissue and incubating same in an ethanol solution;
(ii) incubating the collagen-containing tissue from step (i) in a first solution comprising an inorganic salt and an anionic surfactant in order to denature non-collagenous proteins contained therein;
(iii) incubating the collagen-containing tissue produced in step (ii) in a second solution comprising an inorganic acid until the collagen in said material is denatured; and
(iv) incubating the collagen-containing tissue produced in step (iii) in a third solution comprising an inorganic acid with simultaneous mechanical stimulation for sufficient time to enable the collagen bundles in said collagen-containing tissue to align;
wherein the mechanical stimulation comprises applying tension cyclically to the collagen-containing tissue.

In a third aspect the present invention provides a method of repairing anterior cruciate ligament (ACL) tears, partial or complete, comprising:
(i) providing a collagen-containing patch adapted to enclose at least a portion of said ACL;
(ii) contacting said ACL and/or collagen-containing patch with a sensitizer;
(iii) enclosing said ACL in said collagen-containing patch to produce a bioactive chamber; and
(iv) adhering said collagen-containing patch to said ACL without sutures.

It will be appreciated by those skilled in the art that the step of adhering the collagen-containing patch of the present invention to the soft tissue can be any method known in the art including a tissue weld using laser emitted optical energy or radio frequency ("RF") energy to "weld" the tissue. Other non-suture adhering means such as a biocompatible glue such as fibrin glue or a PLA/PLG polymer might be used.

In some embodiments, the collagen-containing patch is adhered to the soft tissue by tissue welding using a laser. The optimal pulse length for the laser will be determined by the skilled user; however, by way of example a 100 µm thick collagen-containing patch can be adhered using 33-600 ms pulses over 1-6 minutes.

### Figures

Figure 1 comprises a scanning electron microscopy showing the surface morphology of a collagen-containing patch as prepared by the method of Example 1. It can be seen that the patch possesses two distinct surface: a smooth surface featuring compact collagen bundles (A), and a rough, porous surface of loose collagen fibres (B). Scale bar: 500µm.
Figure 2 shows scanning electron microscopy (SEM) image (X100) of a collagen membrane produced by the method of the invention.
Figure 3 shows a "butterfly" arrangement of a patch of the invention.

### Detailed Description Of The Preferred Embodiments Of The Invention

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the specification and relevant art and should not be interpreted in an idealized or overly formal sense unless expressly so defined herein. Well-known functions or constructions may not be described in detail for brevity and/or clarity.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof. As used herein, the term "and/or" includes any and all combinations of one or more of the associated listed items. As used herein, phrases such as "between X and Y" and "between about X and Y" should be interpreted to include X and Y. As used herein, phrases such as "between about X and Y" mean "between about X and about Y". As used herein, phrases such as "from about X to Y" mean "from about X to about Y".

For the recitation of numeric ranges herein, each intervening number there between with the same degree of precision is explicitly contemplated. For example, for the range 6-9, the numbers 7 and 8 are contemplated in addition to 6 and 9, and for the range 6.0-7.0, the numbers 6.0, 6.1, 6.2, 6.3, 6.4, 6.5, 6.6, 6.7, 6.8, 6.9 and 7.0 are explicitly contemplated.

The term "about" as used herein refers to a deviation in the value following the term by 10% above or below. For example, reference to about 50 µm thick collagen-containing patch includes ranges between 45 µm and 55 µm i.e. 10% below or above the 50 µm value. This includes 45 µm, 46 µm, 47 µm, 48 µm, 49 µm, 50 µm, 51 µm, 52 µm, 53 µm, 54 µm and 55 µm.

It will be understood that the sequence of operations (or steps) described in the method of the invention is not limited to the order presented in the claims or figures unless specifically indicated otherwise.

In the broadest possible aspect the present invention relates to the repair of soft tissue using a collagen-containing patch.

The term "collagen" as used herein refers to all forms of collagen, including those which have been processed or otherwise modified. Preferred collagens are treated to remove the immunogenic telopeptide regions ("atelopeptide collagen"), are soluble, and will have been reconstituted into fibrillar form. Type I collagen is best suited to most applications involving ligament tissue, tendon tissue, venous tissue or nervous tissue repair. However, other forms of collagen in combination with type I collagen are also useful in the practice of the invention, and are not excluded from consideration here.

The term "patch" refers to a piece or segment of collagen-containing tissue that has been produced by the methods disclosed herein and which can be placed on and/or adhered to a soft tissue defect or site of the soft tissue defect such that the tissue is repaired. The patch can be any geometric shape but is typically substantially planar and may, in position, conform to the shape of underlying or overlying tissue.

The collagen-containing patch preferably has the following properties:
a) pores that interconnect in such a way as to favour tissue integration and vascularisation;
b) biodegradability and/or bioresorbability so that normal tissue ultimately replaces the scaffold;
c) surface chemistry that promotes cell attachment, proliferation and differentiation;
d) strength and flexibility; and
e) low antigenicity.

The collagen-containing patch is typically prepared or manufactured from "collagen-containing tissue" comprising dense connective tissue found in any mammal. The term "collagen-containing tissue" means skin, muscle and the like which can be isolated from a mammalian body that contains collagen. The term "collagen-containing tissue" also encompasses "synthetically" produced tissue in which collagen or collagen containing material has been assembled or manufactured outside a body.

In some embodiments, the collagen-containing tissue is isolated from a mammalian animal including, but not limited to, a sheep, a cow, a pig or a human. In other embodiments, the collagen-containing tissue is isolated from a human.

In some embodiments, the collagen-containing tissue is "autologous", i.e. isolated from the body of the patient in need of treatment.

In some embodiments, the collagen-containing patch will comprise greater than 80% type I collagen. In other embodiments, the collagen-containing patch will comprise at least 85% type I collagen. In still other embodiments the collagen-containing patch will comprise greater than 90% type I collagen.

The collagen-containing patch may be manufactured by any method known in the art; however, one preferred method includes the following steps:
(i) isolating a collagen-containing tissue and incubating the tissue in an ethanol solution;
(ii) incubating the collagen-containing tissue from step (i) in a first solution comprising an inorganic salt and an anionic surfactant in order to denature non-collagenous proteins contained therein;
(iii) incubating the collagen-containing tissue produced in step (ii) in a second solution comprising an inorganic acid until the collagen in said material is denatured; and
(iv) incubating the collagen-containing tissue produced in step (iii) in a third solution comprising an inorganic acid with simultaneous mechanical stimulation for sufficient time to enable the collagen bundles in said collagen-containing tissue to align;
wherein the mechanical stimulation comprises applying tension cyclically to the collagen-containing tissue.

It will be appreciated that any inorganic salt may be used in the first solution as long as it is capable of forming a complex with Lewis acids. In some embodiments, the inorganic salt is selected from the group consisting of trimethylammonium chloride, tetramethylammonium chloride, sodium chloride, lithium chloride, perchlorate and trifluoromethanesulfonate. In other embodiments, the inorganic salt is lithium chloride (LiCl).

While any number of anionic surfactants may be used in the first solution, in some embodiments, the anionic surfactant is selected from the group consisting of alkyl sulfates, alkyl ether sulfates, alkyl sulfonates, and alkyl aryl sulfonates. Particularly useful anionic surfactants include alkyl sulphates such as sodium dodecyl sulphate (SDS).

In some embodiments, the first solution comprises about 1% (v/v) SDS and about 0.2% (v/v) LiCl.

In some embodiments, the inorganic acid in the second solution comprises about 0.5% (v/v) HCl, while the inorganic acid in the third solution comprises about 1% (v/v) HCl.

It will be appreciated by those skilled in the art that the incubation periods in each of the three steps will vary depending upon: (i) the type of collagen-containing tissue; (ii) the type of inorganic salt / acid and/or anionic surfactant; (iii) the strength (concentration) of each inorganic salt / acid and/or anionic surfactant used and (iv) the temperature of incubation. In some embodiments, the incubation period in step (i) is at least 8 hours. In other embodiments, the incubation period in step (ii) is less than 60 minutes, while in other embodiments the incubation period in step (iii) is at least 20 hours.

In some embodiments, the incubation in step (ii) is at about 4°C. In other embodiments, the incubation in step (ii) is undertaken for at least 12 hours.

In some embodiments, the second solution comprises about 0.5% (v/v) HCl.

In some embodiments, the incubation in step (iii) is undertaken for about 30 minutes. In other embodiments, the incubation in step (iii) is undertaken with shaking.

In some embodiments, the third solution comprises about 1% (v/v) HCl solution.

In some embodiments, the incubation in step (iv) is undertaken for about 12 to 36 hours, preferably for about 24 hours. In other embodiments, the incubation in step (iv) is undertaken with shaking.

In some embodiments, the methods of the invention further comprises a neutralization step between step (iii) and step (iv) which comprises incubation of said collagen-containing tissue with about 0.5% (v/v) NaOH.

In some embodiments, the methods of the invention further comprises step (v) which comprises incubating the collagen-containing tissue from step (iv) with acetone and then drying the collagen-containing tissue.

In some embodiments, the methods of the invention further comprises between steps (ii) and (iii) and/or between steps (iii) and (iv) a step of contacting the collagen-containing tissue with glycerol in order to visualise and facilitate the removal of fat and/or blood vessels.

The glycerol maybe contacted with the collagen-containing tissue for any amount of time that will facilitate the removal of fat and/or blood vessels. In some embodiments, the contact time is at least 10 minutes.

In some embodiments, the methods of the invention further comprises between steps (ii) and (iii) and/or between steps (iii) and (iv) a wash step for the collagen-containing tissue. The purpose of the wash step used between steps (ii) and (iii) is to remove denatured proteins. Thus, any wash solution capable of removing denatured proteins can be used. In some embodiments the wash solution used between steps (ii) and (iii) is acetone.

Following the washing with acetone, the collagen-containing tissue is further washed with sterile water.

In some embodiments, the collagen-containing tissue is further washed in a NaOH:NaCl solution. If the collagen-containing tissue is washed with NaOH:NaCl it is then preferably washed with sterile water.

In some embodiments, after step (iv) the collagen-containing tissue is further washed with the first solution.

The term "simultaneous mechanical stimulation" used in the methods described herein refers to the process of stretching the collagen-containing tissue during the chemical processing of the collagen-containing tissue. The collagen-containing tissue may undergo static and/or cyclic stretching. Accordingly, in some embodiments the simultaneous mechanical stimulation may comprise:
(i) stretching of the collagen-containing tissue for a preset period;
(ii) relaxation of the collagen-containing tissue for a preset period; and
(iii) n-fold repetition of steps (i) and (ii), where n is an integer greater than or equal to 1.

If the mechanical stimulation is carried out by stretching the collagen-containing tissue, the collagen-containing tissue is preferably stretched along its long axis.

In some embodiments, the simultaneous mechanical stimulation comprises applying tension cyclically to collagen-containing tissue, wherein the periodicity of the tension comprises a stretching period of about 10 seconds to about 20 seconds and a relaxing period of about 10 seconds, and the strain resulting therefrom is approximately 10%, and the mechanical stimulation continues until the collagen bundles within the collagen-containing tissue are aligned as described herein.

Once produced the collagen-containing tissue comprises collagen fibres or bundles with a knitted structure. The term "knitted structure" as used herein refers to a structure comprising first and second groups of fibres or bundles where fibres or bundles in the first group extend predominately in a first direction and fibres or bundles in the second group extend predominately in a second direction, where the first and second directions are different to each other and the fibres or bundles in the first group interleave or otherwise weave with the fibres or bundles in the second group. The difference in direction may be about 90°.

The collagen-containing tissue made by the preferred methods comprise a "maximum tensile load strength" of greater than 20N. In some embodiments, the collagen-containing tissue of the present invention has maximum tensile load strength greater than 25N, 40N, 60N, 80N, 100N, 120N or 140N.

Further, it is believed that the knitted structure of the embodiments of the collagen-containing tissue provides reduced extension at maximum load of the collagen-containing patch while providing an increase in modulus.

The term "modulus" as used herein means Young's Modulus and is determined as the ratio between stress and strain. This provides a measure of the stiffness of the collagen-containing tissue and/or patch.

In some embodiments the collagen-containing tissue has a modulus of greater than 100 MPa. In other embodiments the collagen-containing tissue has a modulus of greater than 200 MPa, 300 MPa, 400 MPa, or 500 MPa.

The term "extension at maximum load" as used herein means the extension of the collagen-containing tissue at the maximum tensile load strength referenced to the original length of the collagen-containing tissue in a non-loaded condition. This is to be contrast with maximum extension which will be greater.

In some embodiments, the collagen-containing tissue has extension at maximum load of less than 85% of the original length.

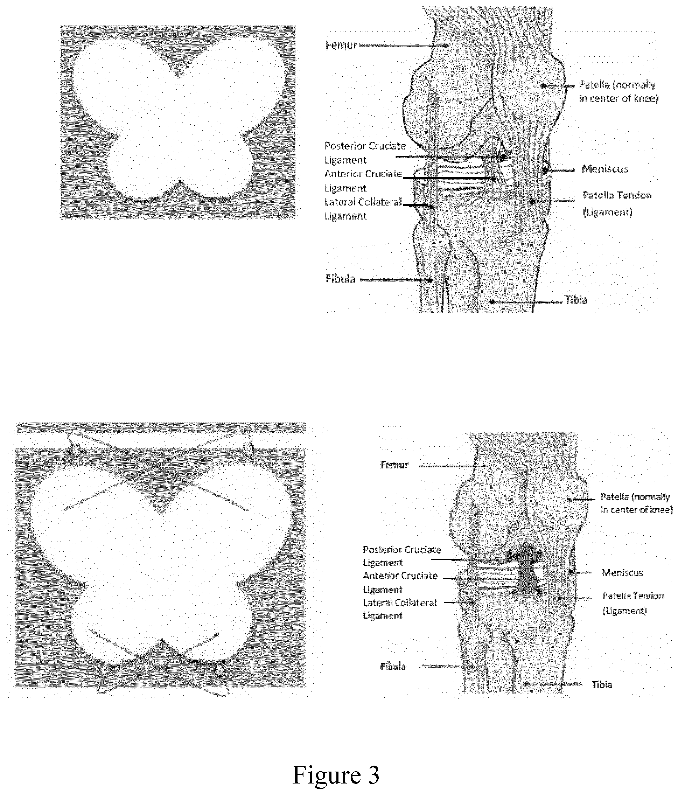

Once the collagen-containing tissue has been produced it may then be shaped into a collagen-containing patch for use. In some embodiments, the collagen-containing patch is adapted by shaping the patch to provide better means of manipulation *in situ.* In some embodiments the collagen-containing patch corresponds to the collagen-containing patch shown in Figure 3.

Most importantly, the collagen-containing patch of the present invention must be sufficiently thick to provide support for the soft tissue under repair; however, not too thick that the ability to adhere the collagen-containing patch to the soft tissue is impaired. Thus, in some embodiments the collagen-containing patch is between 25 µm and 200 µm thick. In some embodiments, the collagen-containing patch is between 30 µm and 180 µm thick. In other embodiments, the collagen-containing patch is between 35 µm and 170 µm thick. In still other embodiments, the collagen-containing patch is between 40 µm and 160 µm thick. In still other embodiments, the collagen-containing patch is between 45 µm and 150 µm thick. In still other embodiments, the collagen-containing patch is between 50 µm and 140 µm thick. In still other embodiments, the collagen-containing patch is between 50 µm and 100 µm thick. Finally, in some embodiments the collagen-containing patch is about 50 µm thick.

Generally stated, embodiments of the subject invention are directed to collagen-containing patch which is particularly suitable for repairing soft tissue defects in mammalian animals or human patients. Other than human the patient can be a primate, an equine, a canine, or a feline animal. Thus, in use, the collagen-containing patch is applied to a soft tissue defect or defect site in a patient to be treated such that it encloses the defect. The term "tissue defect" or "tissue defect site" refers to a disruption of the epithelium or tissue of a tendon, a ligament, a vein/artery or a nerve tunnel. A tissue defect results in a tissue performing at a suboptimal level or being in a suboptimal condition. For example, a tissue defect may be a partial thickness or full thickness tear in a tendon or ligament. A tissue defect can assume the configuration of a "void", which is understood to mean a three-dimensional defect such as, for example, a gap, cavity, hole or other substantial disruption in the structural integrity of the epithelium or tissue. In certain embodiments, the tissue defect is such that it is incapable of endogenous or spontaneous repair. A tissue defect can be the result of accident, disease, and/or surgical manipulation.

The term "enclosing" means that the collagen-containing patch substantially envelops the tissue defect site such that a bioactive chamber is formed beneath the patch and above the tissue defect. The term "bioactive chamber" refers to the space between the collagen-containing patch and the surface of the tissue defect. Within this space endogenous cells or in some embodiments, introduced cells, pharmacologically active agents and the like are contained within the location of the tissue defect thereby enabling cellular and tissue repair.

The terms "repairing" or "repair" or grammatical equivalents thereof are used herein to cover the repair of a tissue defect in a mammalian animal, preferably a human. "Repair" refers to the formation of new tissue sufficient to at least partially fill a void or structural discontinuity at a tissue defect site. Repair does not however, mean or otherwise necessitate, a process of complete healing or a treatment, which is 100% effective at restoring a tissue defect to its pre-defect physiological/structural/mechanical state.

In some embodiments, the collagen-containing patch and/or the tissue defect or defect site is contacted with a sensitizer before or after the collagen-containing patch has been applied to the tissue defect. A sensitizer has the effect of absorbing energy from a radioactive or light ray, and transmitting the energy to the collagen-containing patch and/or soft tissue such that the collagen-containing patch adheres or "welds" to the soft tissue being treated.

Non-exhaustive examples of sensitizers that can be used in the present invention include acetophenones, benzophenones, naphthalenes, biacetyl, eosine, rose bengal, pyrenes, anthracenes, phenothiazines, and the like. In some embodiments the sensitizer is rose bengal (4,5,6,7-tetrachloro-2',4',5',7'-tetraiodofluorescein).

Once the collagen-containing patch has been applied to the tissue defect the skilled artisan such as an orthopaedic surgeon secures the patch at the site of defect in a patient by adhering the patch to the soft tissue defect. Such adherence of the collagen-containing patch to the defect site provides a bioactive chamber as described herein, which seeks to promote cell growth and healing of the tissue under treatment. Examples of the means of adherence include, without limitation, a biocompatible glue, a tissue weld and a combination thereof.

A biocompatible glue may be selected from the group including, but not limited to gelatine, alginic acid, agarose, starch, fibrin, collagen, laminin, elastin, fibroneetin, proteoglycans and/or glycosaminogl yeans, e.g. heparan sulfate, chondroitin sulfate and/or keratan sulfate, casein, dextrans, caramel lose, pectin, carrageen, and xanthan. A non-limiting example of a biocompatible glue that may be used with the present teachings is a fibrin sealant manufactured by Oesterreichisches Institut Fuer Haemoderivate G.M.B.H. in Vienna, Austria and distributed by Baxter Healthcare Corporation, Glendale, Calif. under the brand name TISSEEL™. Non-limiting examples of other adhering means which may be used instead of, or in addition to a biocompatible glue include tissue welds such as described in Helmsworth, T. F., et al., Laser Surgery Medicine 10: 576-583, 1990. Also biocompatible or bioabsorbable material such as, without limitation, a PLA/PLG polymer.

One method the collagen-containing patch may be adhered to the soft tissue defect is by photochemical tissue bonding (PTB), an alternative to light-driven methodologies, which relies on chemical rather than thermal energy to seal the tissue (Chan et al. (2005), J Surg Res; 124: 274-279; Chan et al. (2002) J Surg Res; 108: 77-84; Kamegaya et al. (2005) Lasers Surg Med; 37: 264-270; Mulroy et al. (2000) Invest Ophthalmol Vis Sci; 41: 3335-3340; Proano et al. (2004) J Cataract Refract Surg; 30: 2420-2424; Proano et al. (2004) Invest Ophthalmol Vis Sci; 45: 2177-2181). PTB uses a combination of visible light and a photoreactive dye to create immediate bonds and a tight seal between tissue surfaces. No other additives are required. Absorption of light by the dye results in the generation of reactive intermediates, which induce crosslinking reactions between molecules on the co-apted tissue surfaces (Lambert & Kochevar (1997) Photochem Photobiol., 66: 15-25). Although the exact mechanism of action is unclear it has been demonstrated that Type I collagen can be chemically crosslinked using a photochemical process. PTB occurs by a photochemical mechanism and is typically carried out at much lower powers than laser welding. It does not involve heating of the tissues and may thus provide all the advantages of laser repair while avoiding the sequelae of thermal injury.

Examples of the radioactive ray which may be used in the exposure include far ultraviolet rays such as a bright line spectrum in a mercury lamp (wavelength: 254 nm), a KrF excimer laser (wavelength: 248 nm), an ArF excimer laser (wavelength: 193 nm), an F2 excimer laser (wavelength: 157 nm), and an EUV (wavelength: 13 nm, etc.); X-rays such as synchrotron radioactive rays; charged particle-rays such as electron beams, and the like. The radioactive ray is preferably a far ultraviolet ray and a charged particle-ray. More preferably, the radioactive ray is KrF excimer laser (wavelength: 248 nm), ArF excimer laser (wavelength: 193 nm), F2 excimer laser (wavelength: 157 nm) and electron beams.

Laser light is used to provide uniform illumination to the collagen-containing patch. The laser light is preferably pulsed so that the zone of thermal damage to the nearby soft tissue is minimized. The laser wavelength is chosen so that the light is absorbed by the sensitizer. The energy heats up the thin layer on the patch and the adjacent soft tissue surface, which bonds or "welds" the patch to the soft tissue surface. The patch serves as a means of joining the tissue. The thin layer of sensitizer on the patch minimizes the zone of damage since this layer at the patch-soft tissue interface preferentially absorbs the laser energy and is heated. A thin layer of sensitizer, thus, is preferable to a patch permeated with the sensitizer.

A layer of biocompatible glue may also be present on the patch to facilitate or strengthen the bond, or a chemical that polymerizes upon exposure to the laser light can be used to form a mechanical bond to the soft tissue surface. The layer of sensitizer or biocompatible glue or polymer could also be applied directly to the soft tissue surface in addition to or instead of the coated patch, although coating the patch with the chemical layer may be easier.

The patches or the soft tissue surface typically have a layer of sensitizer or a biocompatible glue, and the patches are welded to the soft tissue defect as laser light is transmitted and absorbed at by the patch.

The pulse structure of the laser light used for patch welding must be chosen carefully to minimize or prevent damage to the soft tissue being welded. The effect of pulsed laser radiation with temperature feedback on endovascular patch welding has been studied using computer simulations. See Glinsky et al., "Computer modeling of endovascular patch welding using temperature feedback", Proceedings of Medical Applications of Lasers III, Vol. 2623, pp. 349-358 (1995) and Glinsky et al., "Modeling of endovascular patch welding using the computer program LATIS", Proceedings of Laser-Tissue Interaction IV, Vol. 2391, pp. 262-272 (1995). These studies, which are hereby incorporated by reference, show that it is possible to control the zone of damage using pulsed laser irradiation.

The optimal pulse length minimizes the total treatment time needed to weld a patch to the soft tissue defect, while keeping the thickness of the damaged tissue to less than 100 µm.

In various embodiments of the present invention the bioactive chamber created by the adherence of the collagen-containing patch and the soft tissue defect may include one or more pharmacologically active agents. As used herein, "pharmacologically active agent" generally refers to a pharmacologically active agent having a direct or indirect beneficial therapeutic effect upon introduction into a host. Representative examples of pharmacologically active agents that may be suitable for use in the presentive invention include:
Antihypertensives such as hydralazine, minoxidil, captopril, enalapril, clonidine, prazosin, debrisoquine, diazoxide, guanethidine, methyldopa, reserpine, trimethaphan;
Calcium channel blockers such as diltiazem, felodipine, amlodipine, nitrendipine, nifedipine and verapamil;
Antiarrhyrthmics such as amiodarone, flecainide, disopyramide, procainamide, mexiletene and quinidine,
Antiangina agents such as glyceryl trinitrate, erythrityl tetranitrate, pentaerythritol tetranitrate, mannitol hexanitrate, perhexilene, isosorbide dinitrate and nicorandil;
Beta-adrenergic blocking agents such as alprenolol, atenolol, bupranolol, carteolol, labetalol, metoprolol, nadolol, nadoxolol, oxprenolol, pindolol, propranolol, sotalol, timolol and timolol maleate;
Adrenergic stimulants such as adrenaline, ephedrine, fenoterol, isoprenaline, orciprenaline, rimeterol, salbutamol, salmeterol, terbutaline, dobutamine, phenylephrine, phenylpropanolamine, pseudoephedrine and dopamine;
Vasodilators such as cyclandelate, isoxsuprine, papaverine, dipyrimadole, isosorbide dinitrate, phentolamine, nicotinyl alcohol, co-dergocrine, nicotinic acid, glycerl trinitrate, pentaerythritol tetranitrate and xanthinol;
Antiproliferative agents such as paclitaxel, actinomycin D, sirolimus, tacrolimus, everolimus and dexamethasone;
Anticoagulants and thrombolytic agents such as warfarin, dicoumarol, low molecular weight heparins such as enoxaparin, streptokinase and its active derivatives;
Hemostatic agents such as aprotinin, tranexamic acid and protamine;
Analgesics and antipyretics including the opioid analgesics such as buprenorphine, dextromoramide, dextropropoxyphene, fentanyl, alfentanil, sufentanil, hydromorphone, methadone, morphine, oxycodone, papaveretum, pentazocine, pethidine, phenopefidine, codeine dihydrocodeine; acetylsalicylic acid (aspirin), paracetamol, and phenazone;
Immunosuppressants, antiproliferatives and cytostatic agents such as rapomycin (sirolimus) and its analogs (everolimus and tacrolimus);
Non-steroidal anti-inflammatory agents including their racemic mixtures or individual enantiomers where applicable, preferably which can be formulated in combination with dermal and/or mucosal penetration enhancers, such as ibuprofen, flurbiprofen, ketoprofen, aclofenac, diclofenac, aloxiprin, aproxen, aspirin, diflunisal, fenoprofen, indomethacin, mefenamic acid, naproxen, phenylbutazone, piroxicam, salicylamide, salicylic acid, sulindac, desoxysulindac, tenoxicam, tramadol, ketoralac, flufenisal, salsalate, triethanolamine salicylate, aminopyrine, antipyrine, oxyphenbutazone, apazone, cintazone, flufenamic acid, clonixerl, clonixin, meclofenamic acid, flunixin, coichicine, demecolcine, allopurinol, oxypurinol, benzydamine hydrochloride, dimefadane, indoxole, intrazole, mimbane hydrochloride, paranylene hydrochloride, tetrydamine, benzindopyrine hydrochloride, fluprofen, ibufenac, naproxol, fenbufen, cinchophen, diflumidone sodium, fenamole, flutiazin, metazamide, letimide hydrochloride, nexeridine hydrochloride, octazamide, molinazole, neocinchophen, nimazole, proxazole citrate, tesicam, tesimide, tolmetin, and triflumidate;
Antimicrobials including the cephalosporins such as cephalexin, cefoxytin and cephalothin;
Penicillins such as amoxycillin, amoxycillin with clavulanic acid, ampicillin, bacampicillin, benzathine penicillin, benzylpenicillin, carbenicillin, cloxacillin, methicillin, phenethicillin, phenoxymethylpenicillin, flucloxacillin, meziocillin, piperacillin, ticarcillin and azlocillin;
Tetracyclines such as minocycline, chlortetracycline, tetracycline, demeclocycline, doxycycline, methacycline and oxytetracycline and other tetracycline-type antibiotics;
Amnioglycoides such as amikacin, gentamicin, kanamycin, neomycin, netilmicin and tobramycin;
Antifungals such as amorolfine, isoconazole, clotrimazole, econazole, miconazole, nystatin, terbinafine, bifonazole, amphotericin, griseofulvin, ketoconazole, fluconazole and flucytosine, salicylic acid, fezatione, ticlatone, tolnaftate, triacetin, zinc, pyrithione and sodium pyrithione;
Quinolones such as nalidixic acid, cinoxacin, ciprofloxacin, enoxacin and norfloxacin;
Sulphonamides such as phthalysulphthiazole, sulfadoxine, sulphadiazine, sulphamethizole and sulphamethoxazole;
Sulphones such as dapsone;
Other miscellaneous antibiotics such as chloramphenicol, clindamycin, erythromycin, erythromycin ethyl carbonate, erythromycin estolate, erythromycin glucepate, erythromycin ethylsuccinate, erythromycin lactobionate, roxithromycin, lincomycin, natamycin, nitrofurantoin, spectinomycin, vancomycin, aztreonarn, colistin IV, metronidazole, tinidazole, fusidic acid, trimethoprim, and 2-thiopyridine N-oxide; halogen compounds, particularly iodine and iodine compounds such as iodine-PVP complex and diiodohydroxyquin, hexachlorophene; chlorhexidine; chloroamine compounds; and benzoylperoxide;

An efficacious amount of the aforementioned pharmacologically active agent(s) can easily be determined by those of ordinary skill in the art taking into consideration such parameters as the particular pharmacologically active agent chosen, the size and weight of the patient, the desired therapeutic effect, the pharmacokinetics of the chosen pharmacologically active agent, and the like, as well as by reference to well-known resources such as Physicians' Desk Reference™: PDR--52 ed (1998)--Medical Economics 1974.

The foregoing description of preferred embodiments of the invention is presented for purposes of illustration and description and is not intended to be exhaustive or to limit the invention to the precise form disclosed. Many modifications and variations are possible in light of the above teaching.

All patents, patent applications, provisional applications, and publications referred to or cited herein are incorporated by reference in their entirety, including all figures and tables, to the extent they are not inconsistent with the explicit teachings of this specification.

The present application also provides fclaimaspects and embodiments as set forth in the following Statements:
Statement 1. A sutureless method of repairing a defect in a soft tissue selected from the group consisting of ligament tissue and tendon tissue comprising:
   (i) providing a collagen-containing patch adapted to wrap around said soft tissue such that the defect is enclosed, wherein the collagen-containing patch has a modulus of greater than 100 MPa;
   (ii) contacting said soft tissue and/or collagen-containing patch with a sensitizer selected from the group consisting of acetophenones, benzophenones, naphthalenes, biacetyl, eosine, rose bengal, pyrenes, anthracenes and phenothiazines;
   (iii) wrapping said soft tissue with said collagen-containing patch such that the tissue is enclosed and a bioactive chamber beneath the patch and above the tissue defect is produced; and
   (iv) adhering said collagen-containing patch to said soft tissue without sutures by exposing said collagen-containing patch to light from a laser or light source.
Statement 2. A sutureless method according to statement 1, where the collagen-containing patch is produced by a process comprising:
   (i) isolating a collagen-containing tissue and incubating same in an ethanol solution;
   (ii) incubating the collagen-containing tissue from step (i) in a first solution comprising an inorganic salt and an anionic surfactant in order to denature non-collagenous proteins contained therein;
   (iii) incubating the collagen-containing tissue produced in step (ii) in a second solution comprising an inorganic acid until the collagen in said material is denatured; and
   (iv) incubating the collagen-containing tissue produced in step (iii) in a third solution comprising an inorganic acid with simultaneous mechanical stimulation for sufficient time to enable the collagen bundles in said collagen-containing tissue to align;
      wherein the mechanical stimulation comprises applying tension cyclically to the collagen-containing tissue.
Statement 3. A method according to statement 1 or 2, wherein the soft tissue is a ligament tissue.
Statement 4. A method according to statement 3, wherein the defect in the ligament tissue is an anterior cruciate ligament (ACL) tear or rupture.
Statement 5. A method according to any one of statements 1 to 4, wherein the sensitizer is rose bengal.
Statement 6. A method according to any one of statements 1 to 5, wherein the collagen-containing patch comprises greater than 80% type I collagen.
Statement 7. A method according to any one of statements 1 to 6, wherein the collagen-containing patch comprises greater than 90% type I collagen.
Statement 8. A method according to any one of statements 1 to 7, wherein the collagen-containing patch has maximum tensile load strength greater than 25N.
Statement 9. A method according to any one of statements 1 to 8, wherein the collagen-containing patch has extension at maximum load of less than 85% of the original length.
Statement 10. A method according to any one of statements 1 to 9, wherein the collagen-containing patch is between 25 µm and 200 µm thick.
Statement 11. A method according to any one of statements 1 to 9, wherein the collagen-containing patch is about 50 µm thick.
Statement 12. A kit for use in a method according to statement 1 comprising:
   (i) a collagen-containing patch which is between 25 µm and 200 µm thick, comprises greater than 90% type I collagen, has maximum tensile load strength of greater than 100N, has a modulus of greater than 200 MPa and is adapted to wrap around said soft tissue such that the defect is enclosed and a bioactive chamber beneath the patch and above the tissue defect is produced;
   (ii) a sensitizer selected from the group consisting of acetophenones, benzophenones, naphthalenes, biacetyl, eosine, rose bengal, pyrenes, anthracenes and phenothiazines; and
   (iii) instructions for use.
Statement 13. A kit according to statement 12, wherein said patch is produced by:
   (i) isolating a collagen-containing tissue and incubating same in an ethanol solution;
   (ii) incubating the collagen-containing tissue from step (i) in a first solution comprising an inorganic salt and an anionic surfactant in order to denature non-collagenous proteins contained therein;
   (iii) incubating the collagen-containing tissue produced in step (ii) in a second solution comprising an inorganic acid until the collagen in said material is denatured; and
   (iv) incubating the collagen-containing tissue produced in step (iii) in a third solution comprising an inorganic acid with simultaneous mechanical stimulation for sufficient time to enable the collagen bundles in said collagen-containing tissue to align;
      wherein the mechanical stimulation comprises applying tension cyclically to the collagen-containing tissue.
Statement 14. A method of repairing an anterior cruciate ligament (ACL), comprising:
   (i) providing a collagen-containing patch adapted to wrap around said ACL so that the ACL or a portion of said ACL is enclosed and wherein the collagen-containing patch has a modulus of greater than 100 MPa;
   (ii) contacting said ACL and/or collagen-containing patch with a sensitizer selected from the group consisting of acetophenones, benzophenones, naphthalenes, biacetyl, eosine, rose bengal, pyrenes, anthracenes and phenothiazines;
   (iii) wrapping said ACL with said collagen-containing patch such that the ACL or a portion thereof is enclosed and a bioactive chamber beneath the patch and above the ACL is produced; and
   (iv) adhering said collagen-containing patch to said ACL without sutures by exposing said collagen-containing patch to light from a laser or light source.
Statement 15. A method according to any one of statements 1 to 11 or 14, wherein the collagen-containing patch is exposed to green light at 532 nm at an irradiance of ∼0.5 W/cm² for 5 minutes to adhere the patch.

Following are examples that illustrate procedures for practicing the invention. These examples should not be construed as limiting.

### Example 1 Method For The Manufacture Of Collagen Membrane

A collagen segment from porcine inner organ lining was carefully separate and placed into a solution comprising about 70% ethanol and allowed to briefly incubate at room temperature. The collagen-containing tissue was then stretched fatty side up over the working surface and as much fat tissue and blood vessels as possible was removed.

In order to visualize fat tissue present the collagen-containing tissue was coated with glycerol for about 10 minutes. At which point the collagen was transparent, but the fat tissue was a white colour. Using forceps we separated the white fat tissue from the collagen under an anatomical microscope.

When complete, the collagen-containing tissue was carefully transferred to a sealed container and incubated in a solution comprising about 1% (v/v) SDS and 0.2% (v/v) LiCl in order to denature the non-collagenous proteins. The incubation was left overnight at 4°C.

The collagen-containing tissue was then carefully washed two times in 100% acetone to remove the denatured the non-collagenous proteins. The tissue was then centrifuged at 100 RPM in a 200 ml container in order to gently spin down residual solutions, non-collagenous proteins and nucleic acids from the collagen-containing tissue.

The collagen-containing tissue was carefully removed and once again washed in membranes Steripure™ water 3 times.

Sometimes, we also washed the collagen-containing tissue in a solution comprising NaOH:NaCl after which we centrifuged the tissue at 100 RPM for 90 minutes.

The collagen-containing tissue was then immersed in 0.5% (v/v) HCl and placed on shaker for 30 minutes to denature the collagen. We found that the concentration of HCl and incubation time was important in order to avoid damaging the mechanical structure of the resulting tissue.

The collagen-containing tissue was then removed and once again washed in Steripure™ water 3 times.

The collagen-containing tissue was then neutralized using 0.5% (v/v) NaOH. At this stage preliminary testing of the mechanical properties of resulting collagen-containing tissue could be undertaken.

The collagen-containing tissue was then manipulated using mechanical forces (compression and extension) using a stainless steel frame. Once the collagen-containing tissue was stretched to the right size, thickness and the like, the tissue was denatured *in situ* i.e. within the frame, immersion in a solution comprising 1% (v/v) HCl. Typically, the tissue was incubated with shaking at 100 RPM for 22-25 hours until the collagen fibre bundles had aligned.

The collagen-containing tissue was then washed with water and rinsed with mixture of 1% (v/v) SDS and 0.2% (v/v) LiCl.

Depending upon the end use, the collagen-containing tissue was then re-coated with glycerol for 10 minutes to visualise any residual fat tissue. As above, forceps were used to separate the remaining white fat tissue from the collagen under an anatomical microscope. Any extra collagen bundles are also removed at this stage in order to control the thickness of the collagen-containing tissue.

Finally, the collagen-containing tissue was treated with acetone and air-dried while still stretched within the frame so that the aligned collagen bundles became fixed. The collagen-containing tissue was then stretched, compressed and/or rolled to create a smooth surface. The finished collagen membrane tissue was then examined and cut to size using a laser cutter.

SEM was performed to characterize the surface morphology of the collagen membrane compared to other types of membranes. In brief, the tissue samples were sputter-coated with 5nm thick platinum (SEM coating unit, E 1020, Hitachi Science Systems Ltd., Japan) and both sides were viewed under a scanning electron microscope (S260, Leica, Cambridge, England) at a low voltage (20 kV).

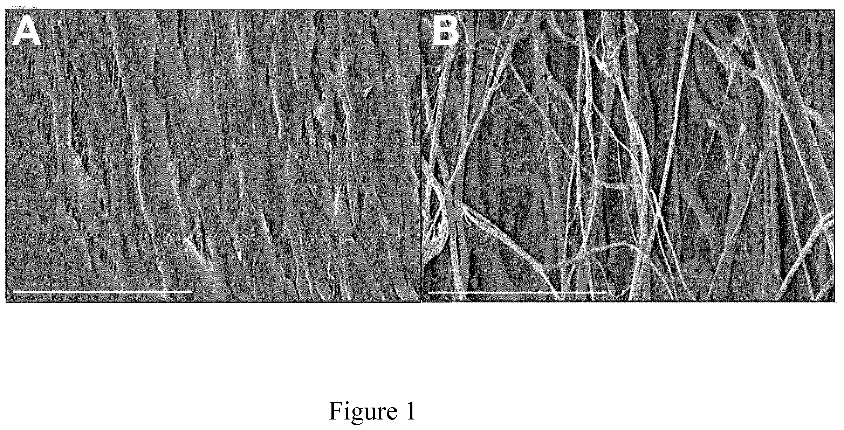

Figure 1 shows the surface morphology of the collagen membrane produced by the methods of the present invention (Tympacol™ referred to as ACS herein) compared to other membranes. Scanning electron microscopy shows the surface morphology of three scaffolds (Panel A-C; ×500, D; ×200). Tympacol™ (referred to as ACS in Figure 1) possesses two distinct surfaces, a smooth surface featuring compact collagen bundles (Panel A), and a rough, porous surface of loose collagen fibres (Panel B). Paper patch (membrane) surface is uneven with few small pores (Panel C). Gelfoam® shows substantial pores of varying sizes (Panel D). Scale bar: 500µm.

Figure 2 shows scanning electron microscopy (SEM) image (X100) of a collagen membrane produced by the above method.

### Example 2 Tendon Repair

Fifty Albino New Zealand White (*Oryctolagus cuniculus*) rabbits between 12 to 20 weeks old with body weight between 3-5kg are used. All rabbits are bred from an out bred rabbit colony maintained in the animal house facility of the University of Western Australia (Nedlands, Australia). Rabbits are fed *ad libitum* with rabbit/guinea pig pellets, hay, and provided water *ad libitum.* Rabbits are held in cages measuring 1.5m wide, 0.75m long, and 0.75m high, with grid floors to prevent pod dermatitis. All operative procedures and cage activities were conducted under strict guidelines detailed by the National Health and Medical Research Council (NHMRC, Canberra, Australia).

The fifty rabbits are randomly allocated into two groups of twenty-five and anaesthetized by intramuscular injection of Ketamine (Parke-Davis, Auckland, NZ) and Xylazine (Troy Laboratories Australia). A longitudinal incision over the left shoulder is made and surgical exposure of the rotator cuff tendon is achieved by releasing a portion of the trapezius and deltoid muscles from the acromion. The rotator cuff tendon is severed thereby creating a soft tissue defect. Then the defect is repaired using either (A) simple suturing of the severed ends of the tendon or (B) the methods described herein. Briefly, a solution of 0.1% (w/v) Rose Bengal (Aldrich, Milwaukee, WI) in phosphate buffered saline is carefully applied to approximately 5 mm of the outer surface of severed tendon. A 50 µm thick collagen-containing patch made by the method of Example 1 as shown in Figure 3 is wrapped around the two ends of the tendon such that the ends of the tendon abut (touch) and such that the defect is enclosed. The patch is then exposed to green light at 532 nm from a 350 mW Compass 415 continuous wave, frequency doubled Nd/YAG laser (Coherent, Inc., Santa Clara, CA, ∼1 cm spot size) at an irradiance of ∼0.5 W/cm² for 5 minutes. This system is chosen because the excitation wavelength is strongly absorbed by Rose Bengal. Other laser parameters may be determined from *ex vivo* pilot studies. Following tendon reconstruction, the wound was closed in layers and dressed, but the limb was not splinted.

At both 4 and 8 weeks, rabbits are anaesthetized and sacrificed by intravenous injection of pentobarbitone. The humerus head, cuff tendon and part of the muscle are harvested from both shoulders (operated and unoperated). Under slack condition, the length and width of both the operated and unoperated tendon are measured with a vernier caliper and the thickness was measured with a micrometer. Samples are then fixed in 4% paraformaldehyde. After fixation, the specimens are decalcified with 10% formic acid, dehydrated, paraffin-embedded, cut to 5µm sections and stained with hematoxylin and eosin and Alcian Blue.

## Claims

1. A sensitizer selected from the group consisting of acetophenones, benzophenones, naphthalenes, biacetyl, eosine, rose bengal, pyrenes, anthracenes and phenothiazines for use in the treatment of a soft tissue defect, comprising:
(i) providing a collagen-containing patch adapted to wrap around said soft tissue such that the defect is enclosed, wherein the collagen-containing patch has a modulus of greater than 100 MPa
(ii) contacting said soft tissue and/or collagen-containing patch with said sensitizer;
(iii) wrapping said soft tissue with said collagen-containing patch such that the tissue is enclosed and a bioactive chamber beneath the patch and above the tissue defect is produced; and
(iv) adhering said collagen-containing patch to said soft tissue without sutures by exposing said collagen-containing patch to light from a laser or light source.

2. A sensitizer for use according to claim 1, where the collagen-containing patch is produced by a process comprising:
(i) isolating a collagen-containing tissue and incubating same in an ethanol solution;
(ii) incubating the collagen-containing tissue from step (i) in a first solution comprising an inorganic salt and an anionic surfactant in order to denature non-collagenous proteins contained therein;
(iii) incubating the collagen-containing tissue produced in step (ii) in a second solution comprising an inorganic acid until the collagen in said material is denatured; and
(iv) incubating the collagen-containing tissue produced in step (iii) in a third solution comprising an inorganic acid with simultaneous mechanical stimulation for sufficient time to enable the collagen bundles in said collagen-containing tissue to align;
wherein the mechanical stimulation comprises applying tension cyclically to the collagen-containing tissue.

3. A sensitizer for use according to claim 1 or 2, wherein the soft tissue is a ligament tissue.

4. A sensitizer for use according to claim 3, wherein the defect in the ligament tissue is an anterior cruciate ligament (ACL) tear or rupture.

5. A sensitizer for use according to any one of claims 1 to 4, wherein the sensitizer is rose bengal.

6. A sensitizer for use according to any one of claims 1 to 5, wherein the collagen-containing patch comprises greater than 80% type I collagen, preferably greater than 90% type I collagen.

7. A sensitizer for use according to any one of claims 1 to 6, wherein the collagen-containing patch has maximum tensile load strength greater than 25N.

8. A sensitizer for use according to any one of claims 1 to 7, wherein the collagen-containing patch has extension at maximum load of less than 85% of the original length.

9. A sensitizer for use according to any one of claims 1 to 8, wherein the collagen-containing patch is between 25 µm and 200 µm thick, preferably about 50 µm thick.

10. A kit comprising:
(i) a collagen-containing patch which is between 25 µm and 200 µm thick, comprises greater than 90% type I collagen, has maximum tensile load strength of greater than 100N, has a modulus of greater than 200 MPa and is adapted to wrap around said soft tissue such that the defect is enclosed and a bioactive chamber beneath the patch and above the tissue defect is produced; and
(ii) a sensitizer selected from the group consisting of acetophenones, benzophenones, naphthalenes, biacetyl, eosine, rose bengal, pyrenes, anthracenes and phenothiazines.

11. A kit according to claim 10, wherein said patch is produced by:
(i) isolating a collagen-containing tissue and incubating same in an ethanol solution;
(ii) incubating the collagen-containing tissue from step (i) in a first solution comprising an inorganic salt and an anionic surfactant in order to denature non-collagenous proteins contained therein;
(iii) incubating the collagen-containing tissue produced in step (ii) in a second solution comprising an inorganic acid until the collagen in said material is denatured; and
(iv) incubating the collagen-containing tissue produced in step (iii) in a third solution comprising an inorganic acid with simultaneous mechanical stimulation for sufficient time to enable the collagen bundles in said collagen-containing tissue to align;
wherein the mechanical stimulation comprises applying tension cyclically to the collagen-containing tissue.

12. A sensitizer for use according to any one of claims 1 to 9, wherein the collagen-containing patch is exposed to green light at 532 nm at an irradiance of ∼0.5 W/cm² for 5 minutes to adhere the patch.
